# EUROPEAN PATENT SPECIFICATION

(11) **EP 1 475 055 B1**
(45) Date of publication and mention of the grant of the patent: **28.04.2010**
(21) Application number: 03010308.9
(22) Date of filing: 07.05.2003
(51) Int. Cl.: A61F 2/16

(54) **Telescopic intraocular lens**
Teleskopintraocularlinse
Lentille intraoculaire téléscopique

(43) Date of publication of application: 10.11.2004
(73) Proprietor: Visioncare Ophthalmic Technologies, Inc., Saratoga, California 95070 (US)
(72) Inventor: Gross, Yosef, Mazor, 73160 (IL); Dotan, Gideon, Yehud, 56275 (IL); Lipshitz, Isaac, Herzelia Pituach, 46448 (IL); Aharoni, Eli, Rishon le Zion, 75302 (IL)
(74) Representative: Modiano, Micaela Nadia

(56) References cited:
- EP-A- 0 162 573
- EP-A- 1 092 402
- EP-A- 1 438 930
- WO-A-00/38593
- US-A1- 2001 018 612
- US-B1- 6 596 026

## Description

The present invention relates generally to intraocular lens (IOL) implants and particularly to a telescopic intraocular lens with novel, compact structure.

### BACKGROUND OF THE INVENTION

Intraocular lens (IOL) inserts comprising telescopes are known. Representative examples of telescopic IOLs include applicant/assignee's US Patents 5,354,335; 5,391,202; 5,814,103; 5,876,442; 5,928,283; 6,007,579 and 6,066,171. Telescopic IOLs may be classified as Galilean, reverse Galilean, or telediopter. Galilean intraocular inserts have a positive (converging) lens at the anterior side of the eye and a negative (diverging) lens at the posterior side. Conversely, reverse Galilean intraocular inserts have a negative (diverging) lens at the anterior side of the eye and a positive (converging) lens at the posterior side.

Galilean telescopic IOLs are designed to correct problems stemming from central field defects, such as those caused by macular degeneration (e.g., atrophic or exudative), chorioretinitis of the macula, central serous chorioretinopathy, or ischemia, for example. Reverse Galilean telescopic IOLs are designed to correct problems stemming from peripheral field defects, such as those caused by retinitis pigmentosa, primary or metastatic central nervous system tumors or glaucoma, for example.

WO 00 38593 discloses a telescopic intraocular lens implant with anti-stare structure for reducing or eliminating the stray light outside the optical path of the telescope, thereby reducing or eliminating glare to the wearer of the IOL and maintaining contrast between perceived figures.

EP 1438930 discloses a telescopic intraocular lens implant with a diffractive lens.

### SUMMARY OF THE INVENTION

The present invention seeks to provide further improvements to telescopic IOLs, as disclosed in claim 1. In one aspect of the present invention, the distance between the positive lens and negative lens is fixed and determined by the structure of the lenses abutting against each other. This novel structure saves on assembly and manufacturing costs.

In another aspect of the present invention, the lenses may be provided with different coatings for superior performance. For example, a yellow coating may be provided to improve night vision. A UV coating may be provided to protect against UV by day. A spectral coating may be provided to improve contrast. An anti-reflective coating may be provided to reduce reflections on the IOL.

In still another aspect of the present invention, the anterior and/or posterior faces of the IOL may be slanted with respect to a longitudinal axis (i.e., the anterior-posterior axis). This means that either the anterior or posterior faces (or both) are prismatic. Such a structure allows for "dialing" the IOL, i.e., rotating the IOL about the longitudinal axis in order to adjust the alignment of the IOL to suit the particular patient. Additionally or alternatively, the IOL is provided with a magnet, and a magnetic tool can be used to dial the IOL non-invasively by attracting the IOL magnet and appropriately turning the IOL.

In yet another aspect of the present invention, the IOL has a truncated cone shape, with one of the lenses being smaller in diameter than the other. Such a structure saves on material, volume and weight, and permits inserting the IOL with a smaller incision.

In another aspect of the present invention, the lenses of the IOL are packaged as separate "capsules" or housings which are aligned and joined, such as by bonding or snap-fitting together.

Different kinds of lenses may be used in the present invention, such as hologramic, graded index, diffractive, binary, multiorder diffractive, harmonic diffractive, Fresnel, spheric and aspheric.

There is thus provided in accordance with a preferred embodiment of the present invention an intraocular lens implant as currently claimed in claim 1.

In accordance with a preferred embodiment of the present invention at least one of the lenses is coated with at least one of a yellow coating, a UV coating, a spectral coating, and an anti-reflective coating.

Further in accordance with a preferred embodiment of the present invention the telescope body has an anterior face, a posterior face and a longitudinal axis, and at least one of the anterior and posterior faces are slanted with respect to the longitudinal axis of the telescope body.

Still further in accordance with a preferred embodiment of the present invention a magnet is mounted on a portion of the implant. Preferably a magnetic tool is provided to attract the magnet from outside an eye in which the implant is installable.

In accordance with a preferred embodiment of the present invention the telescope body has a truncated cone shape with one end having a greater diameter than an opposite end thereof.

Further in accordance with a preferred embodiment of the present invention one of the lenses is smaller than the other, and the smaller of the lenses is positioned near the smaller diameter end of the truncated cone shaped telescope body.

There is also provided in accordance with a preferred embodiment of the present invention an intraocular lens implant including a positive lens mounted in a first housing, a negative lens mounted in a second housing, the first and second housings being aligned and joined together to define an optical path for light to pass therethrough, and mounting structure connected to at least one of the first and second housings for mounting the implant in an eye.

In accordance with a preferred embodiment of the present invention the first and second housings are bonded together. Alternatively, the first and second housings are snap-fit together.

There is also provided in accordance with a preferred embodiment of the present invention an intraocular lens implant including a telescope body defining an optical path for light to pass therethrough, the telescope body having end caps which substantially seal the telescope body, at least one lens attached inside the telescope body, and an air bubble substantially sealed inside the telescope body between the at least one lens and one of the end caps.

In accordance with a preferred embodiment of the present invention a plurality of the lenses are attached inside the telescope body, and at least one other air bubble is substantially sealed inside the telescope body between the lenses. Preferably the telescope body is made of glass.

### BRIEF DESCRIPTION OF THE DRAWINGS

The present invention will be understood and appreciated more fully from the following detailed description, taken in conjunction with the drawings in which:
Figs. 1A and 1B are simplified pictorial and partially sectional illustrations, respectively, of an intraocular lens implant constructed and operative in accordance with the present invention, wherein a distance between a positive lens and a negative lens is fixed and determined by the structure of the lenses abutting against each other;
Fig. 2 is a simplified partially sectional illustration of an intraocular lens implant constructed and operative in accordance with the present invention, wherein the posterior face of the IOL is slanted with respect to a longitudinal axis;
Fig. 3 is a simplified partially sectional illustration of an intraocular lens implant constructed and operative in accordance with yet another preferred embodiment of the present invention, wherein the IOL has a truncated cone shape;
Fig. 4 is a simplified partially sectional illustration of an intraocular lens implant constructed and operative in accordance with another preferred embodiment of the present invention, wherein lenses of the IOL are packaged as separate "capsules" which are aligned and joined together; and
Figs. 5A and 5B are simplified sectional illustrations of two different kinds of diffractive lenses useful in the intraocular lens implants of Figs. 1-4, constructed and operative in accordance with the preferred embodiments of the present invention.

### DETAILED DESCRIPTION OF A PREFERRED EMBODIMENT

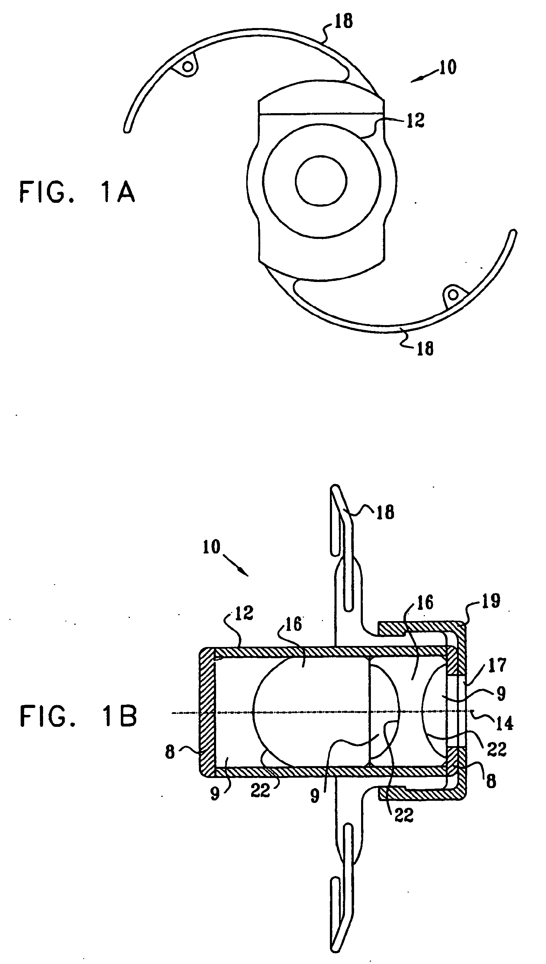

Reference is now made to Figs. 1A and 1B which illustrate an intraocular lens (IOL) implant 10 constructed and operative in accordance with the present invention. IOL implant 10 includes a telescope body 12 defining an optical path 14 for light to pass therethrough. Telescope body 12 is preferably generally cylindrical and includes two lenses 16, attached thereto. One of lenses 16 is positive and the other negative. In the illustrated embodiment, the positive lens is anteriorly positioned and the negative lens is posteriorly positioned, thereby forming a Gaussian telescopic system. It is appreciated that the lenses can be alternatively arranged to form a reverse Galilean telescopic system (having an anteriorly positioned negative lens and a posteriorly positioned positive lens).

Mounting structure 18 is provided for mounting IOL implant 10 in an eye (not shown). Mounting structure 18 may include, for example, one or more haptics extending from telescope body 12. A suitable material for constructing all parts of IOL implant 10, as is well known in the art, is polymethylmethacrylate (PMMA), for example.

In accordance with the present invention, lenses 16 abut against each other. The distance between the two lenses 16 is fixed and determined by the lenses abutting against each other. Lenses 16 are preferably formed with chamfered and flattened surfaces 20 to facilitate lenses 16 abutting against each other. The lenses 16 may be joined to the inside of telescope body 12 by any suitable means, such as by bonding or welding, for example. An optical blocker 19 is snapped onto the posterior end of telescope body 12. Optical blocker 19 preferably includes a translucent or opaque, generally cup-like cap with a generally centrally-located aperture 17 formed therein for passing light therethrough.

In accordance with a preferred embodiment of the present invention, telescope body 12 is made of glass with sealed end caps 8, preferably welded thereto. (The anterior end cap 8 is preferably formed with a generally centrally-located aperture for passing light therethrough.) In such a structure, there is an air bubble 9 between lenses 16 and between each of lenses 16 and each end cap 8. The light is refracted by air bubbles 9 as it passes through telescope body 12. Glass is the preferred material, because it makes a substantially hermetic seal, thereby ensuring the long-term presence and integrity of air bubbles 9.

In accordance with a preferred embodiment of the present invention, one or both of lenses 16 is provided with a coating 22 for superior performance. For example, coating 22 may be yellow to improve night vision. Alternatively, coating 22 may be a UV coating to protect against UV by day. As another alternative, coating 22 may be a spectral coating to improve contrast. Alternatively, coating 22 may be anti-reflective to reduce reflections on IOL implant 10.

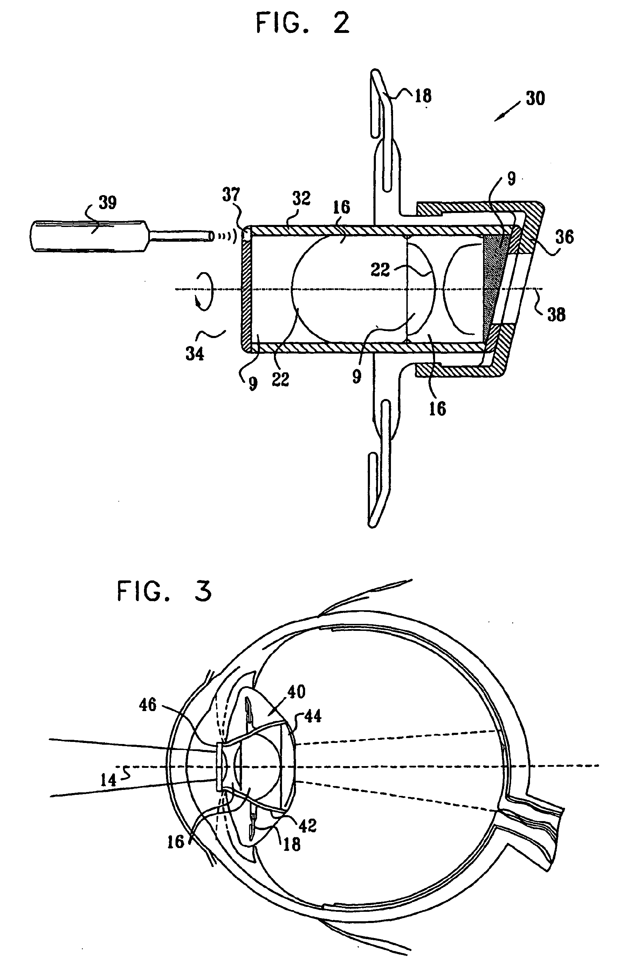

Reference is now made to Fig. 2 which illustrates an intraocular lens implant 30 constructed and operative in accordance with another embodiment of the present invention. Implant 30 is preferably constructed similarly to implant 10, with like elements being designated by like numerals. Implant 30 includes a telescope body 32 that has an anterior face 34, a posterior face 36 and a longitudinal axis 38 (i.e., anterior-posterior axis). Implant 30 differs from implant 10 in that in implant 30, either or both of anterior and posterior faces 34 and 36 are slanted with respect to axis 38. This means that either anterior or posterior faces 34 or 36 (or both) are prismatic. Such a structure allows for "dialing" IOL implant 30, i.e., rotating the IOL about axis 38 in order to adjust the alignment of IOL implant 30 to suit the particular patient. Additionally or alternatively, implant 30 may be provided with a magnet 37 mounted on any part of the structure of implant 30, such as telescope body 32, mounting structure 18 (e.g., the haptics), or even a portion of lenses 16. A magnetic tool 39 can be used to dial implant 30 non-invasively by attracting magnet 37 and appropriately turning implant 30 about axis 38.

Reference is now made to Fig. 3 which illustrates an intraocular lens implant 40 constructed and operative in accordance with yet another embodiment of the present invention. Implant 40 is constructed similarly to implant 10 or implant 30, with like elements being designated by like numerals. Implant 40 includes a telescope body 42 that has a truncated cone shape with one end 44 (in the illustration, the posterior end) having a greater diameter than an opposite end 46 thereof (in the illustration, the anterior end). In this embodiment, one of the lenses 16 (in the illustration, the anterior, negative lens) is preferably smaller than the other (in the illustration, the posterior, positive lens), and the smaller of the lenses is positioned near the smaller diameter end of the truncated cone shaped telescope body 42. Such a structure saves on material, volume and weight, and permits inserting implant 40 with a smaller incision.

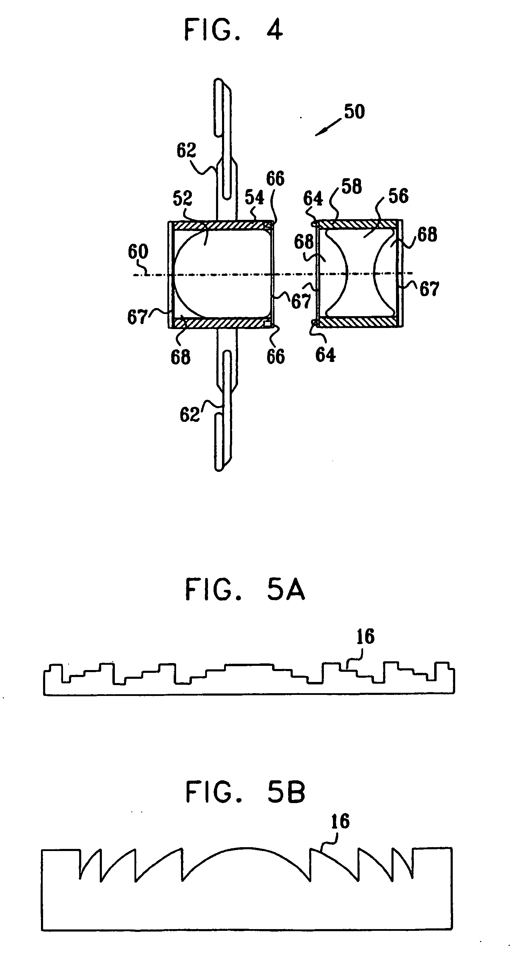

Reference is now made to Fig. 4 which illustrates an intraocular lens implant 50 constructed and operative in accordance with another embodiment of the present invention. IOL implant 50 preferably includes a positive lens 52 mounted in a first housing 54, and a negative lens 56 mounted in a second housing 58. First and second housings 54 and 58 (or "capsules") are aligned and joined together to define an optical path 60 for light to pass therethrough. Mounting structure 62 is preferably connected to first 54 and/or second housing 58 for mounting implant 50 in an eye. First and second housings 54 and 58 may be bonded together. Alternatively, first and second housings 54 and 58 may be snap-fit together, such as by means of male and female snap connectors 64 and 66, respectively, attached to or integrally formed with housings 54 and 58. First and second housings 54 and 58 together comprise a telescope body.

First and second housings 54 and 58 are preferably made of glass with sealed end caps 67 and air bubbles 68 between the end caps and the lenses, as similarly described hereinabove with reference to Fig. 1B.

It is noted that an IOL implant may be constructed in accordance with the present invention, including any combination of the embodiments shown and described above with reference to Figs. 1-4, in so far as they come within the scope of the claims.

In addition, any of the embodiments of the present invention may be made with different kinds of lenses 16, such as holographic, graded index, diffractive, binary, multiorder diffractive, harmonic diffractive, Fresnel, spheric and aspheric. Multiorder lenses are discussed in various optical texts and articles, such as Dean Faklis and G. Michael Morris, "Spectral Properties of Multiorder Diffractive Lenses", Applied Optics, Vol. 34, No. 14, 10 May 1995, particularly pages 2462-2474.

Two examples of diffractive lenses are shown in Figs. 5A and 5B. Fig. 5A illustrates a binary, 4 level lens with a step phase shift of 2π/4, η=80% and power ≈ λ. Fig. 5B illustrates a harmonic Fresnel lens with a phase shift of m2π, η=100% and power ≈ λ. Of course, these are just two examples of the many kinds of diffractive lenses possible within the scope of the invention.

It will be appreciated by persons skilled in the art that the present invention is not limited by what has been particularly shown and described hereinabove. Rather the scope of the present invention includes both combinations and subcombinations of the features described hereinabove as well as modifications and variations thereof which would occur to a person of skill in the art upon reading the foregoing description and which are not in the prior art.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. An intraocular lens implant (10, 30, 40, 50) comprising:
a telescope body (12) defining an optical path (14) for light to pass therethrough; a positive lens (16) and a negative lens (16) attached to said telescope body (12); and
mounting structure (18) connected to said telescope body (12) for mounting said implant (10) in an eye, wherein said lenses (16) abut against each other, and a distance between said positive lens (16) and said negative lens (16) is fixed and determined by said lenses (16) abutting against each other, at least one of said lenses (16) comprises at least one of a diffractive lens, a binary lens, a multiorder diffractive lens, a harmonic diffractive lens, a Fresnel lens, a spheric lens and an aspheric lens, wherein said implant also comprises an optical blocker (19), separate from said mounting structure and operative to snap-fit onto said telescope body (12, 32, 42) and to retain said mounting structure (18) connected thereto.

2. The intraocular lens implant (10) according to claim 1, wherein at least one of said lenses (16) is coated with at least one of a yellow coating, a UV coating, a spectral coating, and an anti-reflective coating.

3. The intraocular lens implant (30) according to any of the preceding claims, wherein said telescope body (32) has an anterior face (34), a posterior face (36) and a longitudinal axis (38), and at least one of said anterior and posterior faces (34, 36) are slanted with respect to said longitudinal axis (38) of said telescope body (32).

4. The intraocular lens implant (30) according to any of the preceding claims, and further comprising a magnet (37) mounted on a portion of said implant (30).

5. The intraocular lens implant (30) according to claim 4 and further comprising a magnetic tool (39) operative to attract said magnet (37) from outside an eye in which said implant (30) is installable.

6. The intraocular lens implant (40) according to any of the preceding claims, wherein said telescope body (42) has a truncated cone shape with one end (44) having a greater diameter than an opposite end (46) thereof.

7. The intraocular lens implant (40) according to claim 6, wherein one of said lenses (16) is smaller than the other, and the smaller of the lenses (16) is positioned near the smaller diameter end (46) of the truncated cone shaped telescope body (42).

8. An intraocular lens implant (50) according to any of the preceding claims wherein lens (16) are either positive ones (52) or negative ones (56), the positive lens (52) being mounted in a first housing (54), the negative lens (56) being mounted in a second housing (58), said first and second housings (54, 58) are aligned and joined together to define an optical path (60) for light to pass therethrough; and said mounting structure (18) being connected to at least one of said first and second housings (54, 58).

9. The intraocular lens implant (50) according to claim 8, wherein said first and second housings (54. 58) are bonded together.

10. The intraocular lens implant (50) according to claim 8 or claim 9, wherein said first and second housings (54, 58) are snap-fit together.

11. An intraocular lens implant (50) according to any of the preceding claims, said telescope body (12) having end caps (67) which substantially seal said telescope body (12), at least one of said lenses (16) being attached inside said telescope body (12), the implant also comprising an air bubble (9, 68) substantially sealed inside said telescope body (12) between said at least one lens (16) and one of said end caps (67).

12. The intraocular lens implant (50) according to claim 11 and further comprising a plurality of said lenses (16) attached inside said telescope body (12), and at least one other air bubble (9, 68) substantially sealed inside said telescope body (12) between said lenses (16).

13. The intraocular lens implant (50) according to claim 11 or claim 12 wherein said telescope body (12) is made of glass.

## Patentansprüche

1. Ein Intraokularlinsen-Implantat (10, 30, 40, 50), das Folgendes umfasst:
einen Teleskopkörper (12), der einen optischen Pfad (14) bestimmt, damit Licht hindurch dringen kann,
eine positive Linse (16) und eine negative Linse (16), die am Teleskopkörper (12) angebracht sind, und
eine Anbringungsstruktur (18), die mit dem Teleskopkörper (12) verbunden ist, zum Anbringen des Implantats (10) in ein Auge, wobei die Linsen (16) aneinander anstoßen und ein Abstand zwischen der positiven Linse (16) und der negativen Linse (16) fest ist und von den Linsen (16) bestimmt wird, die aneinander anstoßen; wobei mindestens eine der Linsen (16) umfasst mindestens eines von Folgendem: einer Beugungslinse, einer Doppellinse, einer Mehrordnungs-Beugungslinse, einer harmonischen Beugungslinse), einer Fresnelschen Linse, einer sphärischen Linse und einer asphärischen Linse, wobei das Implantat auch eine optische Sperre (19) umfasst, die separat von der Anbringungsstruktur ist und wirksam ist, um mit einer Schnappverbindung auf dem Teleskopkörper (12, 32, 42) befestigt zu werden und die damit verbundene Anbringungsstruktur (18) zu halten.

2. Das Intraokularlinsen-Implantat (10) gemäß Anspruch 1, wobei mindestens eine der Linsen (16) mit mindestens einer von Folgendem beschichtet ist: einer gelben Beschichtung, einer UV-Beschichtung, einer Spektral-Beschichtung und einer Antireflex-Beschichtung.

3. Das Intraokularlinsen-Implantat (30) gemäß einem beliebigen der obigen Ansprüche, wobei der Teleskopkörper (32) eine vordere Fläche (34), eine hintere Fläche (36) und eine Längsachse (38) hat und die vordere und/oder die hintere Fläche (34, 36) in Bezug zu der Längsachse (38) des Teleskopkörpers (32) geneigt ist.

4. Das Intraokularlinsen-Implantat (30) gemäß einem beliebigen der obigen Ansprüche, das weiter einen Magneten (37) umfasst, der auf einen Abschnitt des Implantats (30) montiert ist.

5. Das Intraokularlinsen-Implantat (30) gemäß Anspruch 4, das weiter ein magnetisches Werkzeug (39) umfasst, welches wirksam ist, um den Magneten (37) von außerhalb eines Auges anzuziehen, in das das Implantat (30) implantiert werden kann.

6. Das Intraokularlinsen-Implantat (40) gemäß einem beliebigen der obigen Ansprüche, wobei der Teleskopkörper (42) eine Kegelstumpfform hat und das eine Ende (44) einen größeren Durchmesser hat als ein gegenüberliegendes Ende (46) davon.

7. Das Intraokularlinsen-Implantat (40) gemäß Anspruch 6, wobei eine der Linsen (16) kleiner ist als die andere und die kleinere der Linsen (16) nahe dem Ende (46) des kegelstumpfförmigen Teleskopkörpers (42) mit kleinerem Durchmesser positioniert ist.

8. Ein Intraokularlinsen-Implantat (50) gemäß einem beliebigen der obigen Ansprüche, wobei die Linsen (16) entweder positive Linsen (52) oder negative Linsen (56) sind und die positive Linse (52) in einem ersten Gehäuse (54) montiert ist und die negative Linse (56) in einem zweiten Gehäuse (58) montiert ist, wobei das erste und das zweite Gehäuse (54, 58) ausgerichtet sinf und miteinander verbunden sind, um einen optischen Pfad (60) zu bestimmen, damit Licht hindurch dringen kann, und wobei die Anbringungsstruktur (18) mit dem ersten und/oder dem zweiten Gehäuse (54, 58) verbunden ist.

9. Das Intraokularlinsen-Implantat (50) gemäß Anspruch 8, wobei das erste und das zweite Gehäuse (54, 58) miteinander verbunden sind.

10. Das Intraokularlinsen-Implantat (50) gemäß Anspruch 8 oder Anspruch 9, wobei das erste und das zweite Gehäuse (54, 58) durch eine Schnappverbindung miteinander verbunden sind.

11. Ein Intraokularlinsen-Implantat (50) gemäß einem beliebigen der obigen Ansprüche, wobei der Teleskopkörper (12) Endkappen (67) hat, die den Teleskopkörper (12) im Wesentlichen abdichten, wobei mindestens eine der Linsen (16) in dem Teleskopkörper (12) angebracht ist, wobei das Implantat auch eine Luftblase (9, 68) umfasst, die innerhalb des Teleskopkörpers (12) zwischen der mindestens einen Linse (16) und einer der Endkappen (67) im Wesentlichen abgedichtet ist.

12. Das Intraokularlinsen-Implantat (50) gemäß Anspruch 11, das weiter eine Vielzahl der Linsen (16) umfasst, die innerhalb des Teleskopkörpers (12) angebracht sind und mindestens eine andere Luftblase (9, 68), die innerhalb des Teleskopkörpers (12) zwischen den Linsen (16) im Wesentlichen abgedichtet ist.

13. Das Intraokularlinsen-Implantat (50) gemäß Anspruch 11 oder Anspruch 12, wobei der Teleskopkörper (12) aus Glas besteht.

## Revendications

1. Implant sous forme de lentille intraoculaire (10, 30, 40, 50), comprenant :
un corps (12) télescopique, définissant un chemin optique (14) pour la lumière, de manière à lui permettre de passer à travers lui ;
une lentille positive (16) et une lentille négative (16), attachées audit corps (12) télescopique ; et
une structure de montage (18), connectée audit corps (12) télescopique, pour monter ledit implant(10) dans un oeil, dans lequel lesdites lentilles viennent en butée l'une contre chaque autre, et une distance, entre ladite lentille positive (16) et ladite lentille négative (16), est fixée et déterminée par lesdites lentilles (16) venant en butée l'une contre chaque autre, au moins l'une desdites lentilles (16) comprenant au moins l'une d'une lentille diffractive, une lentille binaire, une lentille diffractive dans plusieurs ordres, une lentille diffractive harmonique, une lentille de Fresnel, une lentille sphérique et une lentille asphérique, dans lequel ledit implant comprend également un bloqueur optique (19), séparé de ladite structure de montage et susceptible de fonctionner pour se monter, par clipsage, sur ledit corps (12, 32, 42) télescopique et de retenir ladite structure de montage (18) lui étant connectée.

2. Implant sous forme de lentille intraoculaire (10) selon la revendication 1, dans lequel au moins l'une desdites lentilles (16) est revêtue d'un parmi un revêtement jaune, un revêtement contre les UV, un revêtement spectral, et un revêtement anti-réfléchissant.

3. Implant sous forme de lentille intraoculaire (20) selon l'une quelconque des revendications précédentes, dans lequel ledit corps (32) télescopique comprend une face antérieure (34), une face postérieure (36) et un axe longitudinal (38), et au moins l'une, desdites faces antérieure et postérieure (34, 36) sont inclinées par rapport audit axe longitudinal (38) dudit corps (32) télescopique.

4. Implant sous forme de lentille intraoculaire (30) selon l'une quelconque des revendications précédentes, et comprenant en outre un aimant (37), monté sur une partie dudit implant (30).

5. Implant sous forme de lentille intraoculaire (30) selon la revendication 4, et comprenant en outre un outil magnétique (39), susceptible de fonctionner pour attirer ledit aimant (37), à partir de l'extérieur d'un oeil dans lequel ledit implant (30) est susceptible d'être installé.

6. Implant sous forme de lentille intraoculaire (40) selon l'une quelconque des revendications précédentes, dans lequel ledit corps (42) télescopique présente une forme tronconique, dont une extrémité (44) présente un plus grand diamètre qu'une extrémité (46) opposée de celui-ci.

7. Implant sous forme de lentille intraoculaire (40) selon la revendication 6, dans lequel l'une desdites lentilles (16) est plus petite que l'autre, et la plus petite des lentilles (16) est positionnée à proximité de l'extrémité (46) à plus petit diamètre du corps (42) télescopique présentant une forme tronconique.

8. Implant sous forme de lentille intraoculaire (50) selon l'une quelconque des revendications précédentes, dans lequel les lentilles (16) sont soit des lentilles positives (52), soit des lentilles négatives (56), la lentille positive (52) étant montée dans un premier boîtier (54), la lentille négative (56) étant montée dans un deuxième boîtier (58), lesdits premier et deuxième boîtiers (54, 58) sont alignés et reliés ensemble, de manière à définir un chemin optique (60) pour la lumière, de manière à lui permettre de passer à travers eux ; et ladite structure de montage (18) étant connectée à au moins l'un des premier et deuxièmes boîtiers (54, 58).

9. Implant sous forme de lentille intraoculaire (50) selon la revendication 8, dans lequel lesdits premier et deuxièmes boîtiers (54, 58) sont reliés ensemble.

10. Implant sous forme de lentille intraoculaire (50) selon la revendication 8 ou la revendication 9, dans lequel lesdits premier et deuxièmes boîtiers (54, 58) sont clipsés ensemble.

11. Implant sous forme de lentille intraoculaire (50) selon l'une quelconque des revendications précédentes, ledit corps (12) télescopique comprenant des couvercles d'extrémité (67), fermant de manière sensiblement étanche ledit corps (12) télescopique, au moins l'une desdites lentilles (16) étant attachée à l'intérieur dudit corps (12) télescopique, l'implant comprenant également une bulle d'air (9, 68), enfermée de manière sensiblement étanche à l'intérieur dudit corps (12) télescopique, entre ladite au moins une lentille (16) et l'un desdits couvercles d'extrémité (67).

12. Implant sous forme de lentille intraoculaire (50) selon la revendication 11 et comprenant en outre une pluralité desdites lentilles (16), attachées à l'intérieur dudit corps (12) télescopique, et au moins une autre bulle d'air (6, 68), enfermée de manière sensiblement étanche à l'intérieur dudit corps (12) télescopique, entre lesdites lentilles (16).

13. Implant sous forme de lentille intraoculaire (50) selon la revendication 11 ou la revendication 12, dans lequel ledit corps (12) télescopique est réalisé en verre.
